(19) **Europäisches Patentamt**
**European Patent Office**
**Office européen des brevets**

(11) **EP 4 696 303 A1**

(12) **EUROPEAN PATENT APPLICATION**

(43) Date of publication:
**18.02.2026 Bulletin 2026/08**

(51) International Patent Classification (IPC):
**A61K 9/16** (2006.01)     **A61K 9/20** (2006.01)

(21) Application number: **24194645.8**

(22) Date of filing: **14.08.2024**

(52) Cooperative Patent Classification (CPC):
**A61K 9/1623; A61K 9/1652; A61K 9/1664;**
**A61K 9/1694;** A61K 9/2077

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Bonutra AG**
**6312 Steinhausen (CH)**

(72) Inventors:
• **Specht, Felix**
  **65510 Hünstetten (DE)**
• **Yunis, Mahmud**
  **65510 Hünstetten (DE)**

(74) Representative: **Uexküll & Stolberg**
**Partnerschaft von**
**Patent- und Rechtsanwälten mbB**
**Beselerstraße 4**
**22607 Hamburg (DE)**

(54) **ALL NATURAL GRANULATE**

(57)     The present disclosure relates to a method of producing a granulate based on all-natural ingredients, a granulate obtainable by said method, and its use as a tableting excipient and for the production of other oral solid dosage forms such as capsules having excellent flow properties and excellent compressibility. The method includes the steps of introducing a carrier powder from a plant material into a high-shear mixer granulator, wherein the carrier material exhibits an angle of repose (AoR), as measured according to the fixed funnel method using a plastic funnel with a distance of 7 cm drop height to a 10 cm diameter disk, of at most 45°, preferably of at most 40°, such as between 30° and 45°, preferably between 35° and 40°; introducing a syrup - component (b) - into the mixer while mixing under high shear, where the organic syrup has a loss on drying (LoD) between 10 wt.% and 70 wt%, preferably between 15 wt.% and 30 wt.%, based on the total weight of the syrup; mixing the components in the high shear mixer to produce a granulate.

Fig. 1

**EP 4 696 303 A1**

## Description

FIELD OF THE INVENTION

The present disclosure relates to a method of producing a granulate based on all-natural or organic-certified ingredients, a granulate obtainable by said method, and its use as a tableting excipient and for the production of other oral solid dosage forms such as capsules.

BACKGROUND OF THE INVENTION

Oral solid dosage (OSD) forms are the most popular and preferred by patients due to their ease of administration, efficiency, cost-effectiveness, and shelf stability. Among OSD forms, tablets and capsules are the most preferred.

Tablets are defined as a unit dose of one or more medications contained in solid dosage forms. Tablets are typically designed for oral administration. They continue to be a popular dosage form due to the advantages they offer to both manufacturers and patients. These advantages include simplicity and economy in preparation, stability, and convenience in packaging, shipping, and dispensing. Tablets come in various shapes, with the most common being discoid, although they can also be round, oval, oblong, cylindrical, and triangular. The size and weight of tablets may vary depending on the quantity of drug substance they contain & intended method of administration.

Typical tableting formulations contain, in addition to the active component, so-called tabletting excipients. Usual tablet excipients include e.g. fillers (lactose, cellulose powder, calcium diphosphate, microcrystalline cellulose, sugar alcohols, e.g. mannitol, Sorbitol and Starch), disintegrants (starch (derivatives), croscarmellose, cross-linked PVP, carboxymethyl celullose, lubricants (Stearic acid, magnesium stearate), glidants (silicon dioxide) or mixtures thereof. Tabletting excipients are additives which enable tablets to be manufactured at all in a practical manner and have an important effect on the processability of the tablet formulation and on the properties of the finished tablet. The tabletting excipients are selected depending on the dosage form and on the active components that are used.

In producing a tablet, pharmaceutically active components or dietary supplements are processed together with suitable tabletting excipients in the form of a granulate with the aid of a solvent to form a mixed granulate. Thereafter the mixed granulate is introduced into a tableting machine for it to be compressed in a subsequent step to form a tablet. Tableting formulations which are suitable for direct tabletting should exhibit sufficient plasticity and in particular good flow properties and should not exhibit any segregation tendency. Good plasticity ensures that the tableting formulation can deform under pressure without breaking, which is important for tablet compression.

Hard capsules are another form of the most common solid oral dosage forms and include medication or a nutrition enclosed in an outer shell. This outer shell is broken down in the digestive tract and the medication or nutrition is absorbed into the bloodstream and then distributed and metabolized in much the same way as medication or nutrition from a tablet. Capsule fillings are commonly dry powder blends. The processing and filling of the materials involves a minimum of stress and is one of the reasons why the products are offered in this form. Hard capsules can be filled with formulations that have a wide range of physical properties allowing the formulator to use many different types of excipients to achieve the desired effect. The powder formulations utilized for capsule filling must exhibit optimal flow properties, be non-sticky, and possess sufficient cohesiveness to form plugs at low pressure forces. As in tablets, excellent flow properties are particularly important for uniform filling of the capsule as well as for uniform distribution of the active ingredient within the capsule.

Lactose is a common tableting excipient due to its excellent flowability and good plasticity. For the same reasons, lactose frequently used as an excipient for the manufacture of capsules. Good plasticity is usually achieved by selecting the appropriate lactose crystalline form and/or lactose particle size. During compression, lactose particles undergo plastic deformation, aiding in tablet formation and integrity. In addition hydroxypropyl methylcellulose (HPMC) is often used as an excipient in tablet formulation due to its excellent film-forming properties, controlled release capabilities, and compatibility with various drugs. HPMC is a semisynthetic, inert, viscoelastic polymer. Achieving excellent flowability and good plasticity with HPMC can be challenging due to its inherent properties. Thus, HPMC is often used together with lactose in tableting formulations. US 8 663 684 B2 discloses a method for producing a granulate useful as a tablet excipient such as a filler, wherein the main ingredients of the tablet excipient are lactose and a cellulose derivative, such as HPMC.

Dibasic calcium phosphate anhydrate (DCPA) is another tableting excipient commonly used for producing medicinal and dietary tablets and capsules. It is also known for having exceptional flow and compression characteristics, while also having an ability for rapid disintegration.

In addition lubricants are commonly added to help in the tabletting of many formulations. Lubricants reduce the friction between the tablet and the die metal surface, which reduces the ejection force and helps to ensure that the tablet is cleanly ejected and without cracking or breakage. Magnesium stearate, a metallic salt boundary lubricant, is probably the most commonly used lubricant for pharmaceutical tableting; it is relatively inexpensive, provides high lubrication, has a high melting point, and is chemically stable. Calcium stearate is another metallic salt boundary lubricant that can replace magnesium stearate in a formulation. Magnesium stearate and calcium stearate are produced by the reaction of sodium

2

stearate with magnesium or calcium salts, respectively, and are therefore classified as synthetic compounds.

There is, however, a need to replace synthetic or semisynthetic tablet excipients with all-natural components while maintaining plastic deformability and excellent flow properties. Lactose may also be undesirable in tableting formulations due to a possible lactose intolerance by many patients or consumers. Furthermore, pharmaceutical companies and dietary supplement manufacturers are slowly switching to natural formulations as consumers increasingly look at ingredient lists and do not want to find chemical or synthetic ingredients. Natural excipients are gaining interest worldwide over synthetic excipients because they are non-toxic, biocompatible, less expensive and widely available. Being plant-based, natural or organic excipients are renewable and can be grown and harvested in a sustainable manner, ensuring a constant supply of raw materials.

It is an object of the present invention to provide a method for producing a granulate useful as a tablet excipient which possesses good plastic deformability and in particular excellent flow properties while being produced of all-natural, organic - i.e. non-mineral - components. It is also an object of the invention to provide a granulate useful as a tablet excipient made of all-natural or organic components only, and which possesses the properties above. Finally, it is an object of the invention to provide a method of use of the granulate for producing tablets or a granulate containing a medically active ingredient or a dietary supplement.

SUMMARY OF THE INVENTION

The above-mentioned objects are achieved by the method as claimed in claim 1, by the granulate as claimed in claim 6 and by the method of use as claimed in claim 7. Preferred embodiments are described in the dependent claims.

Disclosed herein is a method for producing a granulate useful for the production of oral solid dosage forms, comprising the steps of:

(a) introducing a carrier powder from a plant material into a high-shear mixer granulator, wherein the carrier material exhibits an angle of repose (AoR) of at most 45°, preferably of at most 40°, such as between 30° and 45°, preferably between 35° and 40°;

(b) introducing a syrup - component (b) - into the mixer while mixing under high shear, where the organic syrup has a loss on drying (LoD) between 10 wt.% and 70 wt%, preferably between 15 wt.% and 30 wt.% such as between 15 wt.% and 25 wt.%, based on the total weight of the syrup;

(c) mixing the components in the high shear mixer to produce a granulate;

(d) removing the so-produced granulate from the mixer; and

(e) optionally drying the granulate.

The angle of repose (AoR) is the constant, three-dimensional angle (relative to the horizontal base) assumed by a cone-like pile of material formed in accordance with the so-called fixed funnel method. In the method for determining the AoR, a plastic funnel is used having a diameter of 10 mm, and a drop height of 10 cm onto a 10 cm diameter disk. The powder is passed through the funnel until the base of the cone-like pile reaches the diameter of the disk.

The loss on drying (LoD) indicates the amount of volatile substances, in particular of water in the samples, and is determined after drying 5.0 g of the sample in an aluminum dish at 105°C $\pm$ 2°C until the sample reaches a constant mass.

In an aspect, the syrup is a natural or at least an organic syrup. In an aspect, the syrup has a viscosity between 100 mPas and 10,000 mPas, preferably between 200 mPas and 3,000 mPas, such as between 250 mPas and 2,000 mPas at a temperature of 25°C. The term, "viscosity", as used herein, refers to the resistance of a fluid to flow. The term, "syrup", as used herein, refers to aqueous solutions of sugars or starch hydrolysates as well as natural syrups such as agave syrup, date syrup and blueberry syrup. Viscosity is expressed in terms of millipascalseconds (mPas) at a given temperature. Brookfield viscometer (model LVDV-E 115, Brookfield Engineering Inc., Middlesboro, Massachusetts) with a 12-ml small sample adapter was employed for the determination of viscosity. Temperature of the small sample adapter was controlled using a circulation water bath. Spindle #S-25 was used while rotation speed was varied so that the percent torque fell between 25% to 75% during the viscosity measurements. As regards measurement of viscosity of syrups, reference is made to EP 2 288 714 A1, the disclosure of which is fully incorporated by reference.

In an aspect, the carrier - component (a) - is a mixture of at least two plant materials, at least one of the plant materials having an having an angle of repose above 45° and at least one of the plant materials having an angle of repose below 45°, provided that the angle of repose of thee mixture of the plant materials has an overall angle of repose of at most 45°, preferably of at most 40°, such as between 30° and 45°, preferably between 35° and 40°.

In an aspect, the carrier - component (a) - is a plant material containing art least 3% by weight silica. In another aspect, the carrier contains at least 10% by weight, such as at least 20% by weight silica. In an aspect, at least one component the plant material is selected from the group consisting of ground rice hulls, rice straw or bamboo extract. As normally rice hulls, rice straw or bamboo extract have an angle of repose higher than 45°, those carrier components are admixed with plant material that has an angle of repose lower than 45°, preferably lower than 40°, and most preferably lower than 35° in an amount sufficient to yield a carrier - component (a) - having an angle within the ranges described above. For example, the other component may be selected from group consisting of rice extract, isomalt, maltodextrin, acacia gum and the like.

According to the present disclosure, a syrup - component (b) - is to be understood as a viscous, concentrated solution of an organic sugar such as sucrose, fructose or galactose or a combination thereof in water. While some syrups such as agave syrup or date syrup may be derived directly from plants and are classified as "natural syrup", others such as isomalt syrup are obtained by mixing natural, sugar containing components with water and are classified as "organic syrup". In an aspect of the invention, the syrup is selected from the group consisting of agave syrup, date syrup, *gum arabic* syrup, isomalt syrup, and a combination of the foregoing.

In an aspect, the weight ratio of component (a) to component (b) in the granulate is adjusted to be in the range of from about 95:5 to about 50:50, preferably from about 90:10 to about 60:40, more preferably from about 80:20 to about 70:30. In particular the weight ratio of component (a) to component (b) in the granulate is adjusted to be about 95:5, 94:6, 93:7, 92:8, 91:9, 90:10, 89:11, 88:12, 87:13, 86:14, 85:15, 84:16, 83:17, 82:18, 81:19, 80:20, 79:21, 78:22, 77:23, 76:24, 75:25, 74:26, 73:27, 72:28, 71:29, 70:30, 69:31, 68:32, 67:33, 66:34, 65:35, 64:36, 63:37, 62:38, 61:39, 60:40, 59:41, 58:42, 57:43, 56:44, 55:45, 54:46, 53:47, 52:48, 51:49, 50:50 and any ratio in between the stated ratios. It has been found that at a weight ratio of component (a) to component (b) below 50:50 such as 46:54, the produced granulate is not free flowing, while at ratios above 95:5 the flowability and plasticity of the granulate will be poor.

In an aspect, the mixing in the high shear mixer takes place at a temperature of from 15°C to 40°C, preferably of from 20°C to 35°C. In an aspect, a high shear mixer is a closed vessel equipped with mixing tools such as an agitator blade and a chopper.

In another aspect the disclosure relates to a granulate obtainable or obtained by the method as described above. According to one aspect, the granulate obtainable or obtained by the method as described above can be characterized by an excellent flowability as shown by a Hausner ratio below 1.20, preferably below 1.18, more preferably below 1.16, still more preferably below 1.14, and most preferably below 1.12. The Hausner ratio is another indicator of flowability derived from the bulk and tapped densities and calculated as follows:

$$H = \rho_T/\rho_B,$$

where $\rho_B$ is the freely settled bulk density of the powder, as determined according to the European Pharmacopeia 07/2010:20934, Method 1, and $\rho_T$ is the tapped density of the powder, as determined according to the European Pharmacopeia 07/2010:20934, Method 1.

A Hausner ratio close to 1 indicates good flowability.

In another aspect, the granulate obtainable or obtained by the method as described above can be characterized as having an excellent flowability shown by a Carr Index of at most 20, preferably of at most 15, more preferably of at most 10.

The Carr Index is an indicator of the compressibility of a powder, and is calculated as follows:

$$C = (\rho_T-\rho_B)/\rho_B*100,$$

where $\rho_B$ is the freely settled bulk density of the powder and $\rho_T$ is the tapped density of the powder as described above.

According to still another aspect, the a granulate obtainable or obtained by the method as described above can be characterized as having both, a Hausner ratio below 1.20, preferably below 1.18, more preferably below 1.16, still more preferably below 1.14, and most preferably below 1.12, and a Carr Index of at most 20, preferably of at most 15, more preferably of at most 10, where any combination of levels of preference between Hausner ratio and Carr Index is within the ambit of this disclosure.

According to still another aspect, obtainable or obtained by the method as described above can be characterized having an angle of repose between 30 and 40, preferably between 30 and 37, more preferably between 31 and 35.

The granulate obtainable or obtained by the method as described above is useful as a carrier or excipient for producing tablets or capsules. In particular, the granulate is useful for producing pharmaceutical or dietary supplement containing tablets or capsules.

BRIEF DESCRIPTION OF THE DRAWINGS

In the accompanying drawings the characteristics and advantages of the products according to the present disclosure are described in more detail, wherein

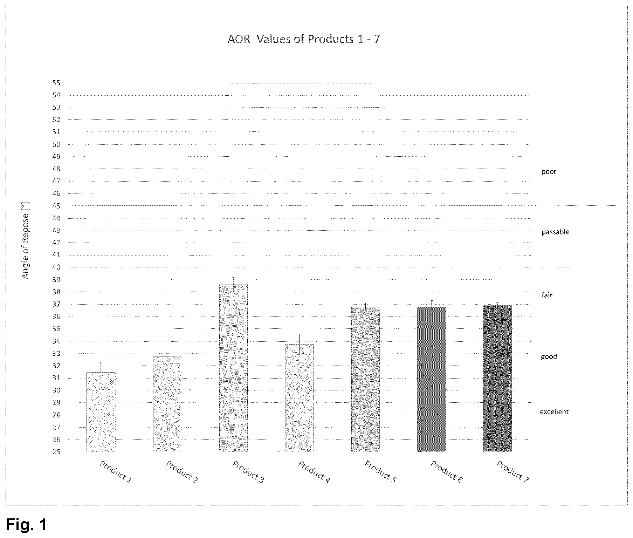

Figure 1 depicts the angle of repose values of several products obtained according to the examples.

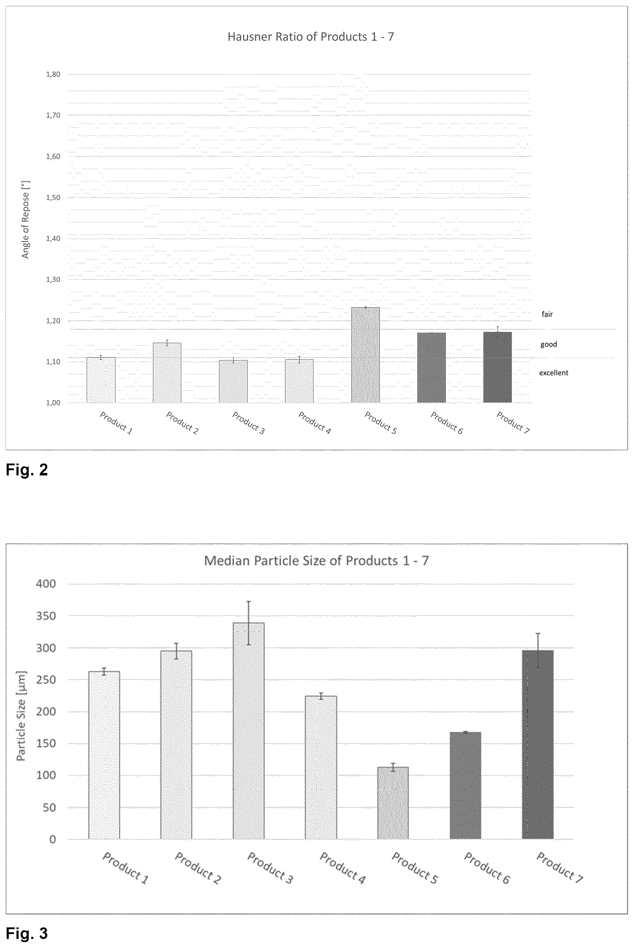

Figure 2 depicts the Hausner ratios of several products obtained according to the examples.

Figure 3 depicts the median particle size in $\mu$m of several products obtained according to the examples.

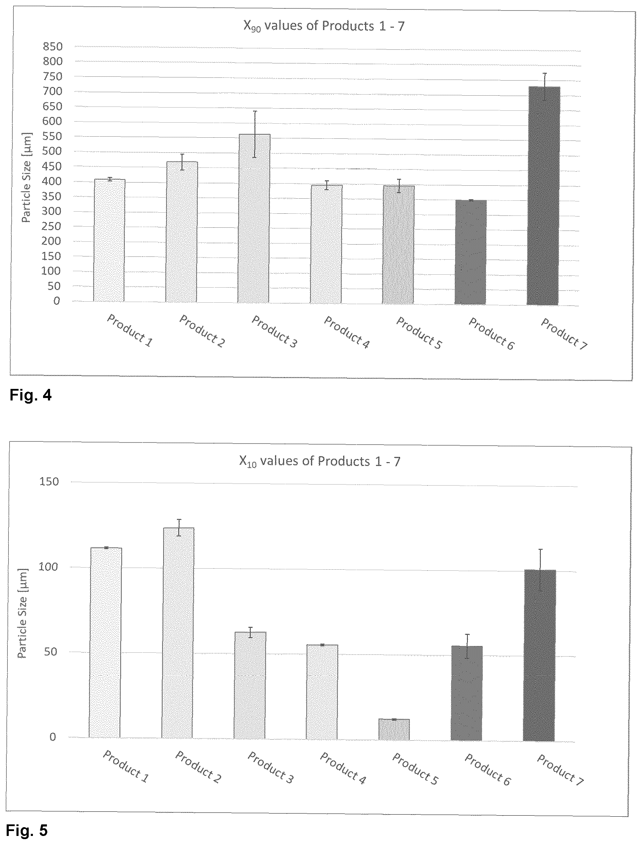

Figure 4 depicts the point in particle size distribution $\chi_{90}$ in $\mu$m of several products obtained according to the examples up to and including which, 90% of the total volume of material in the sample is contained, i.e. 90 % of the number of all particles are smaller or equal to the depicted size.

Figure 5 depicts the point in particle size distribution $\chi_{10}$ in $\mu$m of several products obtained according to the examples up to and including which, 10% of the total volume of material in the sample is contained, i.e. 10 % of the number of all particles are smaller or equal to the depicted size.

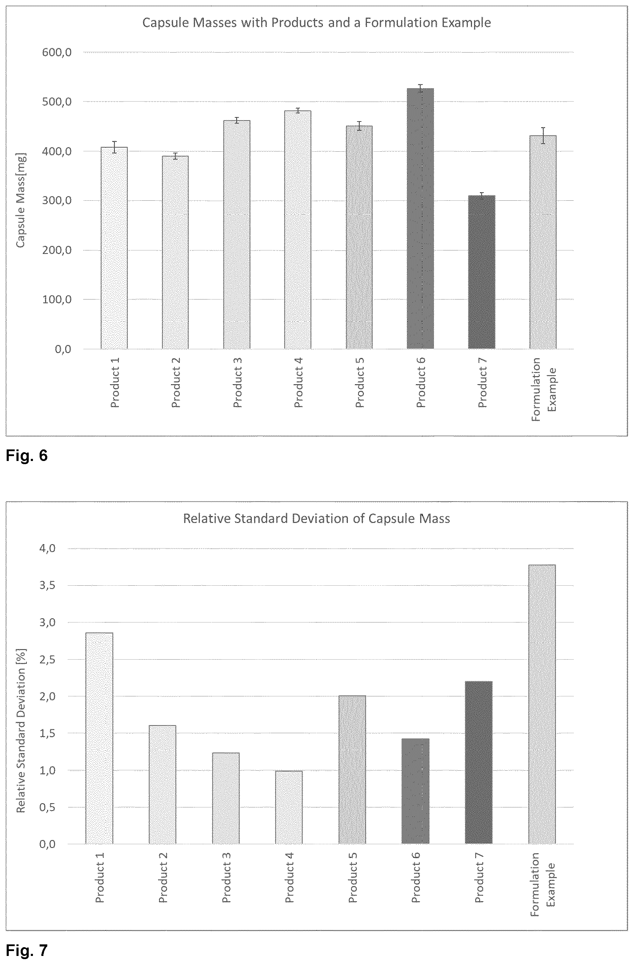

Figure 6 depicts average masses of each of 100 standard size 00 hard gelatine capsules filled with a mixture of the granulated filler according to the description and calcium carbonate (40% w/w), and, further, of 100 standard size 00 hard gelatine capsule filled with calcium carbonate only ("Formulation Example").

Figure 7 depicts the relative standard deviation of average masses of 20 standard size 00 hard gelatine capsules filled with mixture of the granulated filler according to the description and calcium carbonate (40% w/w), and, further, of 20 standard size 00 hard gelatine capsule filled with calcium carbonate only ("Formulation Example"). The uniformity of capsule filling weight was determined by randomly selecting 20 capsules from each batch.

DETAILED DESCRIPTION OF THE INVENTION

In the present disclosure, it has been found that the a granulate obtained by the method according to the present disclosure possesses several properties which ensures the quality, efficacy, and manufacturability of the final product:

Non-reactivity: The granulate obtained by the method of the present disclosure is chemically inert and non-reactive with the active pharmaceutical ingredient (API), the nutritional ingredient or other excipients within the oral dosage form formulation. This maintains stability and efficacy of the drug.

Compressibility: The granulate obtained by the method of the present disclosure exhibits good compressibility, allowing it to form strong, cohesive tablets that do not break apart easily during handling, packaging, or transportation.

Flowability: The granulate obtained by the method of the present disclosure exhibits good flow properties ensuring uniform filling of tablet dies and uniform filling of capsules, which is crucial for consistent tablet weight and content uniformity.

Particle Size: The granulate obtained by the method of the present disclosure exhibits uniform particle size and distribution helping to achieve consistent tablet density and to minimize segregation during the manufacturing process.

Solubility: The granulate obtained by the method of the present disclosure exhibits can be formulated to be appropriately soluble or insoluble in gastrointestinal fluids. Soluble fillers can aid in the dissolution of the active ingredient, while insoluble fillers can be used for controlled-release formulations.

**Safety:** The granulate obtained by the method of the present disclosure is non-toxic, hypoallergenic, and generally recognized as safe (GRAS) by regulatory authorities.

Cost-effectiveness: The granulate obtained by the method of the present disclosure can be produced in a cost-effective manner, as the ingredients are readily available om the market ensuring economic viability of the tablet or capsule formulation.

Compatibility: The granulate obtained by the method of the present disclosure is compatible with various manufacturing processes, such as direct compression, wet granulation, and dry granulation thereby offering flexibility in tablet or capsule production.

The distinction between tablets and capsules can be a significant factor. Tablets are subjected to high compression forces to achieve a solid, compact form with minimal porosity, a quality that is essential for their formation during subsequent handling. In contrast, a hard capsule contains a powdery mass with high porosity, which may or may not undergo compression into a plug, and is encased in a shell capable of withstanding handling.

According to the present disclosure, the granulate is produced by mixing the carrier - component (a) - and a syrup - component (b) - in a high shear mixer. While mixing the powdered ingredients, the syrup is added slowly and on the top of the powder. The process energy generated by the mixer and knifes is considerable, which has the effect of finely grinding the material content into a refined, homogeneous product.

Suitable carrier components - component (a) - include mixtures of rice hull and rice extract, mixtures of rice hull and acacia gum, mixtures of bamboo fiber and rice extract, mixtures of bamboo fiber and acacia gum, mixtures of oat fiber and rice extract, mixtures of oat fiber and acacia gum, mixtures of corn starch and rice extract, mixtrues of corn starch and acacia gum, and any of the foregoing mixtures further including maltodextrin. If desired, also non-organic components can be introduced in the admixture such as calcium hydrogen phosphate. Most preferred are mixtures of rice hull and rice extract. The ratio of the components in the admixture is adjusted to yield an overall angle of repose of at most 45°, preferably of at most 40°, such as between 30° and 45°, preferably between 35° and 40°.

Suitable syrups include agave syrup, date syrup, acacia gum syrup and isomalt syrup.

Suitable compositions of components (a) and (b) include, for example,

1. Rice Hull - 30 to 45 wt.%

   Rice Extract - 27 to 31 wt.%
   Agave Syrup - 30 to 33 wt.%

2. Rice Hull - 30 to 45 wt.%

   Rice Extract - 27 to 31 wt.%
   Date Syrup - 36 to 40 wt.%

3. Rice Hull - 38 to 46 wt.%

   Rice Extract - 31 to 33 wt.%
   Acacia gum syrup - 23 to 28 wt.%

4. Rice Hull - 34 to 42 wt.%

   Rice Extract - 26 to 30 wt.%
   Isomalt syrup - 31 to 37 wt.%

5. Corn Starch - 36 to 40 wt.%

   Acacia gum - 39 to 43 wt.%
   Agave Syrup - 5 to 10 wt.%
   Maltodextrin - 13 to 17 wt.%

6. Calcium hydrogen phosphate - 66 to 72 wt.%

   Acacia gum - 13 to 15 wt.%
   Agave syrup - 2 to 10 wt.%
   Maltodextrin - 13 to 15 wt.%

7. Oat fiber - 35 to 42 wt.%

   Acacia gum - 25 to 32 wt.%
   Agave syrup 28 to 34 wt.%.

High shear mixers are highly effective in achieving an even distribution of particles in the mix. The intensive mixing action ensures that all ingredients are fully mixed, preventing the formation of agglomerates and minimising uneven distribution. The result of the method according to the present invention is a product with consistent particle size distribution, excellent flowability and excellent plasticity. The shaping process for granulation in a high shear mixer - or high shear granulator - takes place in a closed vessel equipped with mixing tools such as an agitator blade and a chopper. The particles are simultaneously pulverized and mixed while the organic syrup - component (b) - is added. Without wishing to be bound to any theory, it is estimated that this creates liquid and solid bridges and very dense granules are formed.

The granulate obtained according to the method described in the present disclosure show an angle of repose of at most 33°, preferably of at most 30°. At the same time, the granulate obtained according to the method described in the present disclosure haven an even particle size distribution and optimal median particle size for capsule filling or direct tablet compression. The granulate obtained according to the method described in the present disclosure have a particle size and particle size distribution that does not excessively promote powder segregation.

EXAMPLES

Particle Size Measurement

The particle size and particle size distribution of the granulates were analysed by laser diffraction using a Helos System (Sympatec GmbH, Clausthal-Zellerfeld, Germany) at 0.5 to 1.5 bar keeping the optical concentration between 0.2 and 0.5.

Bulk Density and Tapped Density Measurement

The bulk density and tapped density of the granulates were analysed using an ERWEKA Tapped Density Tester SVM 122 (Erweka GmbH, Langen, Germany). The Hausner Ratio and the Carr Index were calculated from the bulk density and tapped density values using the formulas described above.

Angle of Repose Measurement

The angle of repose of the granulates were analysed using a in accordance with the so-called fixed funnel method. In the method for determining the AoR, a plastic funnel is used having a diameter of 10 mm, and a drop height of 10 cm onto a 10 cm diameter disk. The powder is passed through the funnel until the base of the cone-like pile reaches the diameter of the disk.

Filling of Capsules Measurement

Six series of capsules were prepared by filling size 00 hard gelatine capsules (Capsugel-Bornem, Belgium) with the granulated filler containing and calcium carbonate (40%). The powder formulations for each series were prepared by weighing and mixing the ingredients. The powder was used for the preparation of 100 capsules with a filling quantity in mg to be assessed. The capsules were filled manually using a capsule filling machine (WEPA Apothekenbedarf GmbH & Co KG, Hillscheid, Germany). Prior to dispensing the next batch of capsules, the machine was cleaned to remove any residual powder particles. "Formulation Example" contains pure calcium carbonate having a measured angle of repose of 52.2° and a measured Hausner ratio of 1.71.

Uniformity of Capsule Filling Measurement

The uniformity of capsule filling weight was determined by randomly selecting 20 capsules from each batch. The powder formulation from the capsules was sampled and accurately weighed individually using an analytical balance (Mettler AT 201, Switzerland). The results were expressed as mean values of 20 determinations in each batch. The calculations of the average weight of the powders were used to determine the standard deviation. Again, the "Formulation Example" contains pure calcium carbonate.

General Preparation Example

A granulate was produced by introducing the carrier powder from a plant material as described in the examples into a high shear mixer. While mixing the powdered ingredients, syrup was added slowly and on the top of the powder. The angles of repose and the Hausner ratios of the individual carrier powders were determined prior to mixing. "AoR" indicates the angle of repose of the individual carrier powders before mixing, and "HR" indicates the Hausner ratio of the individual carrier powders before mixing.

*Product 1*

| Material | Concentration |
|---|---|
| Rice Hull | 39.2 wt.% (AoR 49.5°, HR 1.41) |
| Rice Extract | 29.4 wt.% (AoR 38.1°, HR 1.11) |
| Agave syrup | 31.4 wt.% (LoD 21.94 wt.%; Visc. 968 mPas) |

*Product 2*

| Material | Concentration |
|---|---|
| Rice Hull | 35.3 wt.% (AoR 49.5°, HR 1.41) |
| Rice Extract | 26.5 wt.% (AoR 38.1°, HR 1.11) |
| Date syrup | 38.2 wt.% (LoD 19.32 wt.%) |

*Product 3*

| Material | Concentration |
|---|---|
| Rice Hull | 41.8 wt.% (AoR 49.5°, HR 1.41) |
| Rice Extract | 31.2 wt.% (AoR 38.1°, HR 1.11) |
| Acacia gum syrup | 27.0 wt.% |

*Product 4*

| Material | Concentration |
|---|---|
| Rice Hull | 38.0 wt.% (AoR 49.5°, HR 1.41) |
| Rice Extract | 28.0 wt.% (AoR 38.1°, HR 1.11) |
| Isomalt syrup | 34.0 wt.% (25.11 wt.%) |

*Product 5*

| Material | Concentration |
|---|---|
| Corn starch | 39.5 wt.% (AoR 50.1°, HR 1.18) |
| Acacia gum | 39.5 wt.% (AoR 31.4°, HR 1.22) |
| Agave syrup | 5.2 wt.% (LoD 21.94 wt.%; Visc. 968 mPas) |
| Maltodextrin | 15.8 wt.% (AoR 33.2°, HR 1.18) |

*Product 6*

| Material | Concentration |
| --- | --- |
| Calcium hydrogen phosphate | 68.0 wt.% (AoR 33.1°, HR 1.19) |
| Acacia gum | 13.7 wt.% (AoR 31.4°, HR 1.25) |
| Agave syrup | 4.6 wt.% (LoD 21.94 wt.%; Visc. 968 mPas) |
| Maltodextrin | 13.7 wt.% (AoR 33.2°, 1.18) |

*Product 7*

| Material | Concentration (range) |
| --- | --- |
| Oat Fiber | 39.2 wt.% (AoR 53.0°, HR 1.76) |
| Acacia gum | 29.4 wt.% (AoR 31.4°, HR 1.25) |
| Agave syrup | 31.4 wt.% (LoD 21.94 wt.%; Visc. 968 mPas) |

The angle of repose values of Products 1 to 7 are depicted in Figure 1. It can be seen that Products 1, 2 and 4 exhibit good flow properties according to the determined angles of repose, far below the angles of repose of the individual ingredients, rice hull and rive extract. While rice hull has an angle of repose of 49.5°, and rice extract has an angle of repose of 38.1°, the angle of repose of Product 1 is 31.4°, of Product 2 is 32.9° and of Product 4 is 33.8°. The angle of repose of Product 3 is 38.5° and is still comparable to that of rice extract despite the product containing a large amount of rice hull having a much higher angle of repose. The angle of repose of Products 5, 6 and 7 are all still fair being in the range of from 36.8° to 37.0° despite a very high content of components with a very high angle of repose such as corn starch, calcium hydrogen phosphate and oat fiber, respectively.

The Hausner ratios of Products 1 to 7 are depicted in Figure 2. It can be seen that Products 1, 3 and 4 all have excellent Hausner ratios between 1.10 and 1.11, below the Hausner ratios of the individual carrier components. Also Products 2, 6 and 7 exhibit good Hausner ratios of 1.15, 1.17 and 1.17, respectively, below the Hausner ratio of at least one of the individual carrier components.

As depicted in Figures 3 to 5, all Products 1 to 7 exhibit excellent particle size and particle size distribution to be able of being homogeneously filled in capsules or introduced in to tableting dies in combination with active ingredients or nutritional supplements. This is evident From Figures 6 and 7 showing that capsules can be filled homogeneously with the granulate combined with calcium carbonate (40 wt.%, based on the total weight of the mixture) to yield capsules with consistent weight, even if an active ingredient or a nutritional component, in the present case calcium carbonate in the, is added to the mixture, the calcium carbonate having a measured angle of repose of 52.2° and a measured Hausner ratio of 1.71.

**Claims**

1.  A method for producing a granulate useful for the production of oral solid dosage forms, comprising the steps of:

    (a) introducing a carrier powder from a plant material into a high-shear mixer granulator, wherein the carrier material exhibits an angle of repose (AoR), as measured according to the fixed funnel method using a plastic funnel with a distance of 7 cm drop height to a 10 cm diameter disk, of at most 45°, preferably of at most 40°, such as between 30° and 45°, preferably between 35° and 40°;
    (b) introducing a syrup - component (b) - into the mixer while mixing under high shear, where the organic syrup has a loss on drying (LoD) between 10 wt.% and 70 wt%, preferably between 15 wt.% and 30 wt.% such as between 15 wt.% and 25 wt.%, based on the total weight of the syrup;
    (c) mixing the components in the high shear mixer to produce a granulate;
    (d) removing the so-produced granulate from the mixer; and
    (e) optionally drying the granulate.

2.  The method of claim 1, wherein the weight ratio of component (a) to component (b) in the granulate is adjusted to be in the range of from about 95:5 to about 50:50, preferably from about 90:10 to about 60:40, more preferably from about 80:20 to about 70:30

3. The method of any one of claims 1 or 2, wherein the mixing in the high shear mixer takes place at a temperature of from 15°C to 40°C, preferably of from 20°C to 35°C.

4. The method of any one of claims 1 to 3, wherein the high shear mixing takes place in a closed vessel equipped with an agitator blade and a chopper.

5. The method of any one of claims 1 to 4, wherein the syrup has a viscosity between 100 mPas and 10,000 mPas, preferably between 200 mPas and 3,000 mPas, such as between 250 mPas and 2,000 mPas at a temperature of 25°C.

6. A granulate obtainable by the method of any one of the foregoing claims.

7. The granulate of claim 6, **characterized by** an angle of repose between 30 and 40, preferably between 30 and 37, more preferably between 31 and 35.

8. The granulate of any one of claims 6 or 7, **characterized by** a Hausner ratio below 1.20, preferably below 1.18, more preferably below 1.16, still more preferably below 1.14, and most preferably below 1.12.

9. The granulate of any one of claims 6 to 8, **characterized by** a Carr Index of at most 20, preferably of at most 15, more preferably of at most 10.

10. A use of a granulate obtainable by the method of any one of the foregoing claims for producing a tablet, comprising the steps of

   (a) mixing the granulate obtainable by the method of any one of the foregoing claims with at least active ingredient or a dietary supplement, and optionally with other excipients, and
   (b) introducing the mixture into a tableting machine to produce a mixture.

11. A use of a granulate obtainable by the method of any one of the foregoing claims for filling a tablet, comprising the steps of

   (a) mixing the granulate obtainable by the method of any one of the foregoing claims with at least active ingredient or a dietary supplement, and optionally with other excipients, and
   (b) introducing the mixture into a hard gelatine capsule.

**Fig. 1**

Fig. 2

Fig. 3

**Fig. 4**

**Fig. 5**

**Fig. 6**

**Fig. 7**

Europäisches
Patentamt
European
Patent Office
Office européen
des brevets

# EUROPEAN SEARCH REPORT

**Application Number**

EP 24 19 4645

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 039 813 A (PEPPER TAMMY [GB] ET AL) 21 March 2000 (2000-03-21) * column 3, lines 12-14 - lines 32-42; claims 1, 3-7; examples 1-6; table 1 * ----- | 6,7 | INV. A61K9/16 ADD. A61K9/20 |
| X | CN 101 496 793 A (UNIV TIANJIN [CN]) 5 August 2009 (2009-08-05) * claims 1,2; example 5 * ----- | 6 | |
| X | US 2005/019397 A1 (JACOBS RICHARD L [US]) 27 January 2005 (2005-01-27) * paragraphs [0011], [0015] - paragraph [0016]; claims 1-3, 10-11 * ----- | 1-6,8,9 | |
| X | US 10 350 171 B2 (DEXCEL LTD [IL]; KITOV PHARMACEUTICALS [IL]) 16 July 2019 (2019-07-16) * page 10, column 11, lines 15-25; claim 1; table 3 * ----- | 6,10,11 | |
| T | DE 699 26 012 T2 (GILEAD SCIENCES INC [US]) 4 May 2006 (2006-05-04) * paragraph [0050] * ----- | | **TECHNICAL FIELDS SEARCHED (IPC)** A61K |

The present search report has been drawn up for all claims

| Place of search | Date of completion of the search | Examiner |
|---|---|---|
| The Hague | 16 January 2025 | Munzone, Alessia |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 19 4645

This annex lists the patent family members relating to the patent documents cited in the above-mentioned European search report.
The members are as contained in the European Patent Office EDP file on
The European Patent Office is in no way liable for these particulars which are merely given for the purpose of information.

16-01-2025

| Patent document cited in search report | | Publication date | Patent family member(s) | | Publication date |
|---|---|---|---|---|---|
| US 6039813 | A | 21-03-2000 | AT | E141157 T1 | 15-08-1996 |
| | | | CA | 2058959 A1 | 08-12-1990 |
| | | | DE | 69028107 T2 | 23-01-1997 |
| | | | DK | 0474714 T3 | 02-09-1996 |
| | | | EP | 0474714 A1 | 18-03-1992 |
| | | | ES | 2093027 T3 | 16-12-1996 |
| | | | FI | 892797 A | 08-12-1990 |
| | | | JP | 2770934 B2 | 02-07-1998 |
| | | | JP | H04505918 A | 15-10-1992 |
| | | | KR | 920700616 A | 10-08-1992 |
| | | | NO | 300157 B1 | 21-04-1997 |
| | | | US | 6039813 A | 21-03-2000 |
| | | | WO | 9014821 A1 | 13-12-1990 |
| CN 101496793 | A | 05-08-2009 | NONE | | |
| US 2005019397 | A1 | 27-01-2005 | CA | 2531637 A1 | 03-02-2005 |
| | | | MX | PA06000702 A | 11-04-2006 |
| | | | US | 2005019397 A1 | 27-01-2005 |
| | | | WO | 2005009395 A2 | 03-02-2005 |
| US 10350171 | B2 | 16-07-2019 | CN | 111065382 A | 24-04-2020 |
| | | | US | 2019008777 A1 | 10-01-2019 |
| | | | US | 2019282507 A1 | 19-09-2019 |
| | | | US | 2020390713 A1 | 17-12-2020 |
| | | | WO | 2019008583 A1 | 10-01-2019 |
| DE 69926012 | T2 | 04-05-2006 | AR | 021670 A1 | 31-07-2002 |
| | | | AT | E298576 T1 | 15-07-2005 |
| | | | AU | 759869 B2 | 01-05-2003 |
| | | | BR | PI9916820 A2 | 30-10-2001 |
| | | | CA | 2355239 A1 | 22-06-2000 |
| | | | CN | 1330547 A | 09-01-2002 |
| | | | CN | 1682743 A | 19-10-2005 |
| | | | CO | 5261557 A1 | 31-03-2003 |
| | | | DE | 69926012 T2 | 04-05-2006 |
| | | | EP | 1140114 A2 | 10-10-2001 |
| | | | ES | 2245130 T3 | 16-12-2005 |
| | | | HK | 1040488 A1 | 14-06-2002 |
| | | | ID | 30032 A | 01-11-2001 |
| | | | JP | 4750946 B2 | 17-08-2011 |
| | | | JP | 2002532429 A | 02-10-2002 |
| | | | KR | 20010080765 A | 22-08-2001 |
| | | | MY | 126526 A | 31-10-2006 |
| | | | NZ | 511855 A | 25-07-2003 |
| | | | TR | 200101746 T2 | 21-12-2001 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 24 19 4645

This annex lists the patent family members relating to the patent documents cited in the above-mentioned European search report.
The members are as contained in the European Patent Office EDP file on
The European Patent Office is in no way liable for these particulars which are merely given for the purpose of information.

16-01-2025

| Patent document cited in search report | Publication date | Patent family member(s) | | Publication date |
|---|---|---|---|---|
| | | TW | I230618 B | 11-04-2005 |
| | | WO | 0035460 A2 | 22-06-2000 |

For more details about this annex : see Official Journal of the European Patent Office, No. 12/82

EPO FORM P0459

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8663684 B2
- EP 2288714 A1